## Europäisches Patentamt

## European Patent Office

## Office européen des brevets

(19)

(11) Veröffentlichungsnummer: **0 061 740**
**B1**

(12) ## EUROPÄISCHE PATENTSCHRIFT

(45) Veröffentlichungstag der Patentschrift:
07.01.88

(51) Int. Cl.⁴: **C 12 N 15/00**

(21) Anmeldenummer: **82102559.0**

(22) Anmeldetag: **26.03.82**

(54) Verfahren zur Herstellung von Hybridisierungssonden.

(30) Priorität: 28.03.81 DE 3112338

(43) Veröffentlichungstag der Anmeldung:
06.10.82 Patentblatt 82/40

(45) Bekanntmachung des Hinweises auf die Patenterteilung:
07.01.88 Patentblatt 88/1

(84) Benannte Vertragsstaaten:
AT BE CH DE FR GB IT LI LU NL SE

(56) Entgegenhaltungen:
WO - A - 85/01517
US - A - 4 237 224

JOURNAL OF VIROLOGICAL METHODS, Band 3, no. 1,
Juli 1981, K. VON DER HELM et al.: "Cloning of hepatitis
A virus genome", Seiten 37-43
CHEMICAL ABSTRACTS, Band 94, 1981, Seite 418,
Zusammenfassung 99593w, Columbus, Ohio, US, A.G.
COULEPIS et al.: "Evidence that the genome of
hepatitis A virus consists of single-stranded RNA"
INFECTION AND IMMUNITY (1976) Vol. 13 (3) 898-908
GENETIC ENGINEERING, Principles and Methods Vol. 1
(1979) (J.K. Setlow, A. Hollaender eds) Plenum Press,
N.Y. (USA) pp. 15-36
NATURE (1980) Vol. 288, pp. 236-241
NATURE (1981) Vol. 289, pp. 555-559

(73) Patentinhaber: **Von der Helm, Klaus, Glatzerstrasse 33,
D-8034 Gemering (DE)**
Patentinhaber: **Winnacker, Ernst-L., Elvirastrasse 4,
D-8000 München 19 (DE)**
Patentinhaber: **Deinhardt, Friedrich,
Hermine-Bland-Strasse 9, D-8000 München 90 (DE)**

(72) Erfinder: **Von der Helm, Klaus, Glatzerstrasse 33,
D-8034 Gemering (DE)**
Erfinder: **Winnacker, Ernst-L., Elvirastrasse 4,
D-8000 München 19 (DE)**
Erfinder: **Deinhardt, Friedrich, Hermine-Bland-Strasse 9,
D-8000 München 90 (DE)**

(74) Vertreter: **von Kreisler, Alek, Dipl.-Chem. et al,
Deichmannhaus am Hauptbahnhof, D-5000 Köln 1 (DE)**

**Beschreibung**

Die Erfindung betrifft ein Verfahren zur Herstellung von Hybridisierungssonden zur Selektion von HAV-Komplementär DNA enthaltenden Klonen, die für HAV-Antigene codieren.

Hepatitis A (HA) bleibt eine Krankheit, die im Rahmen der öffentlichen Gesundheit von grosser Bedeutung ist, und ihr Verursacher wurde erst kürzlich identifiziert. Die Arbeitsausfälle sind lang, die Krankheitskosten (durch z.B. Isolierung des Kranken) hoch. Eine passive, besser noch allgemeine aktive Impfung wäre von grossem gesundheitspolitischem und volkswirtschaftlichem Nutzen. Dazu müsste der Erreger dieser Krankheit, das Hepatitis-A-Virus (HAV) in grosser Menge zur Verfügung stehen um a) wissenschaftliche Studien zu treiben, b) das virale Antigen zur Immunisierung benutzen zu können. Das ist augenblicklich nicht der Fall. Virusmaterial steht noch nicht einmal in solcher Menge zur Verfügung, dass eine ausreichende biochemisch-virologische Untersuchung des Viruspartikels durchgeführt werden kann.

Hepatitis A wurde 1967–1970 erstmals auf nichtmenschliche Primaten übertragen (ein Überblick wurde von Deinhardt, F., 1976, in «Advances in Virus Research, Vol. 20, Herausgeber F.B. Bang (Academic Press, New York), S. 113, gegeben), und Viruspartikel wurden später im Stuhl von in der akuten Phase befindlichen Patienten durch Immunelektronenmikroskopie von Feinstone et al 1973 identifiziert (Feinstone, S.M., A.Z. Kapikian und R.H. Purcell, Science 182 (1973) 1026).

Die biophysikalischen und biochemischen Eigenschaften des Hepatitis-A-Virus (HAV) wurden von Provost et al (P.H. Provost, B.S. Wolanski, W.J. Miller, O.L. Ittensohn, W.J. McAleer und M.R. Hilleman, Proc. Soc. Exp. Biol. Med. 148 (1975) 532), Siegl und Frösner (G. Siegl und G.G. Frösner, J. Virol. 26 (1978) 40 und 48), Coulepis et al (A.G. Coulepis, S.A. Locarnini, A.A. Ferris, N.I. Lehmann und I.D. Gust, Intervirology 10 (1978) 24 und A.G. Coulepis, S.A. Locarnini und I.D. Gust, J. Virol. 35 (1980) 572) Bradley et al (D.W. Bradley, C.L. Hornbeck, E.H. Cook und J.E. Maynard, J. Virol. 22 (1978) 228 und D.W. Bradley, H.A. Fields, K.A. McCaustland, E.H. Cook, C.R. Gravelle und J.E. Maynard, J. Med. Virol. 2 (1978) 175), Feinstone et al (S.M. Feinstone, Y. Moritsugu, J.W.K. Shih, J.L. Gerin und R.H. Purcell in «Viral Hepatitis», herausgegeben von G.N. Vyas, S.N. Cohen und R. Schmidt (Abacus Press, Turnbridge Wells) 1979, S. 41), Maynard und Bradley (J.E. Maynard und D.W. Bradley (1980) in «Virus and the Liver», herausgegeben von L. Bianchi, W. Gerock, K. Sickinger und A. Stadler (MTP Press Ltd., Lancaster), S. 9) und Tratschin et al (J.D. Tratschin, G. Siegl, C.G. Frösner und F. Deinhard, 1981, J. Virol., im Druck) bestimmt, und es besteht nun allgemeine Übereinstimmung, dass HAV ein Picornavirus mit den meisten Charakteristiken eines Enterovirus ist.

Das Genom von HAV ist eine einsträngige lineare RNA (wahrscheinlich mit Poly-A-Regionen am 3'-Ende) mit einer Grösse von 2,25 bis $2,8 \times 10^6$ Dalton und einem Sedimentationskoeffizienten von 32,5 bis 33 S im Vergleich zu Poliovirus, das etwa 6500 Nucleotiden entspricht. Die Genkarte von HAV und die Expression der Gene sind unbekannt. Über die Struktur der Genprodukte der HAV-Proteine und ihre Antigenizität ist wenig bekannt.

Obwohl HAV kürzlich in Zellkulturen gezüchtet worden ist (P.H. Provost und M.R. Hilleman, Proc. Soc. Exp. Biol. Med. 160 (1979) 231; G.G. Frösner, F. Deinhardt, R. Scheid, V. Gauss-Müller, N. Holmes, V. Messelberger, G. Siegl und J.J. Alexander, Infection 7 (1979) 303; B. Flehmig, Münch. med. Wschr. 119 (1977) 825 und F. Deinhardt, R. Scheid, V. Gauss-Müller, G.G. Frösner und G. Siegl, Progr. Med. Virol. 27 (1981) im Druck, zeigen infizierte Zellen keinen cytophatischen Effekt (CPE) d.h. sie werden durch die Virusinfektion nicht abgetötet und Viruspartikel werden nicht freigegeben. Jedoch ist der Wachstumszeitraum in der Gewebekultur ungewöhnlich lang, die Ausbeute relativ gering und stark durch die Tatsache behindert, dass das Virus nicht von den Kulturzellen freigesetzt wird, sondern durch künstliche Zerstörung dieser Zellen gewonnen werden muss, wobei die Gefahr der Kontamination von zellulärem Material aussergewöhnlich hoch ist. Die Reinigung von HAV aus solchen Kulturen ist kompliziert, weil infizierte Zellen lysiert und HAV von den Zelltrümmern abgetrennt werden muss.

Es besteht daher ein Bedürfnis, in ausreichender Menge sauberes Virusmaterial des HAV zu erhalten, um HAV-Antigene darzustellen, die eine immunisierende Wirkung gegen HAV aufweisen.

Die Erfindung stellt sich daher die Aufgabe, ein Verfahren zur Herstellung von Hybridisierungssonden, die für die Selektion von HAV-Komplementär DNA enthaltenden Klonen, die HAV-spezifische Antigene exprimieren, verwendet werden können, zur Verfügung zu stellen.

Diese Aufgabe, die sich aus der nachfolgenden Beschreibung ergibt, wird durch die Erfindung gelöst.

Gemäss der Erfindung wird das HAV-Genom in Form einer komplementär DNA (= cDNA) in ein Bakterienplasmid kloniert (eingebaut) die Bakterienklone werden gezüchtet und auf Grund der Hybridisierung mit $^{32}$P-markierten HAV-RNA selektiert. Die Bakterienklone werden auf Expression von HAV-Antigene radioimmunologisch getestet. Die Klone, die auf einem 2- bis 3-fachen cpm Wert über dem negativen Testhintergrund reagieren, werden ausgewählt und können als Hybridisierungssonden zur Selektion von weiteren HAV-cDNA enthaltenden Klonen verwendet werden. Dabei wurde wie folgt verfahren:

Vom Stuhl entnommenes Hepatitis-A-Virus (HAV) wurde stark gereinigt. Die genomische RNA wurde zu cDNA mittels der AMV-reversen Transcriptase transkribiert, und diese DNA wurde dann in den Pst I-Ort des Plasmid pBR 322 kloniert, und Klone wurden in Gegenwart von Tetracyclin selektiert. Die meisten Klone enthielten Einfügungen im Plasmid, die mit HAV-spezifischer RNA hybridisieren, die von HAV-infizierten Zellkulturen isoliert worden war, die von einem menschlichen hepatocellulären Karzinom stammten. Einige Klone zeigten Expression unterschiedlicher Mengen viraler Antigene. Die positiven Klone können als Hybridisierungssonden und Primer für die Herstellung von weiterer cDNA unter Verwendung von RNA von mit HAV infizierten Zellkulturen verwendet werden. Damit ist es möglich, fast unbegrenzte Mengen von viralem Material herzustellen. Weiter ist es möglich, gezielt nur dasjenige Virusmaterial, d.h. die viralen Antigenproteine, herstellen zu können, die zum Immunitätsprozess benötigt werden, nicht aber anderes Virusmaterial, das als Ballast kostenaufwendig ist. Bei dieser Methode des Klonierens wird vermieden, infektiöses Material (Virusgenom) mit herzustellen, das unter grossem Aufwand wieder entfernt oder weggereinigt werden müsste. Die genetische Information für die viralen Antigene bleibt in dem Bakterium verankert, die Bakterien produzieren nur das Immunmaterial.

Die durch das erfindungsgemässe Verfahren erhaltenen Hybridisierungssonden können beispielsweise auch für den diagnostischen Nachweis auf das Vorhandensein von HAV in klinischem Material verwendet werden.

Die Erfindung wurde nachstehend weiter anhand der Abbildungen erläutert.

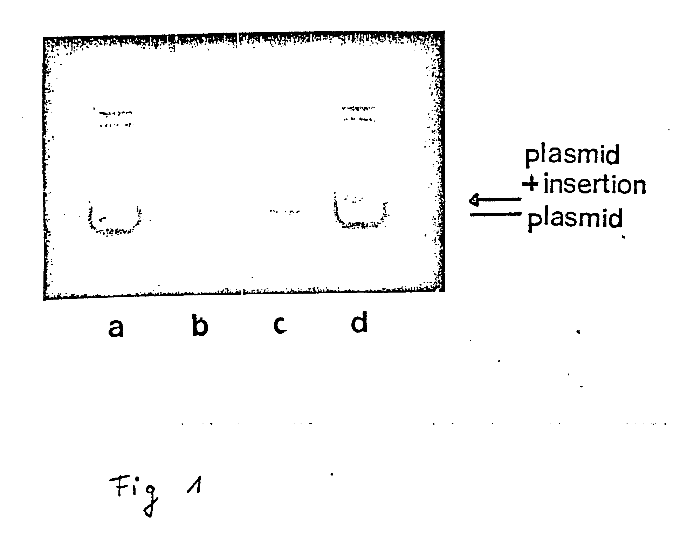

Fig. 1: Grösse der eingefügten DNA

Einzelne Bakterienklone wurden gezüchtet und Plasmide hergestellt. Die Grösse der Plasmide wurde durch Elektrophorese auf 1%igem Agarosegel ermittelt und Banden durch Ethidiumbromid in UV-Licht sichtbar gemacht. Die Aufnahmen 2 und 3 zeigen zwei typische Plasmide mit DNA-Einfügung, und die Aufnahmen 1 und 4 stellen das Bezugsplasmid pBR 322 ohne Einfügung dar.

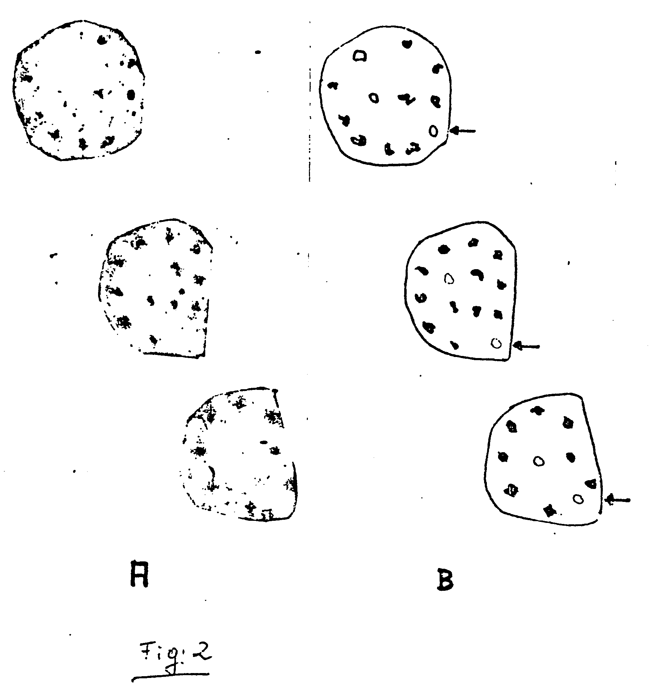

Fig. 2: Hybridisierung von Plasmid-Einfügungen mit $^{32}$P-markierter HAV-RNA

Klone wurden auf Nitrocellulosefiltern über Agar gemäss Grunstein und Hogness (loc.cit.) gezüchtet. HAV-spezifische RNA, gewonnen von mit HAV infizierten PLC/PRF5-Zellen (Alexander et al, 1978, loc. cit.) und mit $^{32}$P markiert, wurde zu den auf Nitrocellulosefiltern gezüchteten Bakterienklonen hybridisiert. A) Autoradiogramm von drei Filtern mit gewachsenen Klonen nach Hybridisierung mit HAV-spezifischer RNA; B) schematische Darstellung dieser drei Filter (die Pfeile unten rechts stellen Kontrollkolonien dar, die pBR 322-DNA ohne Einfügung enthalten); Klone **0** = Hybridisierung positiv; Klone 0 = Hybridisierung negativ.

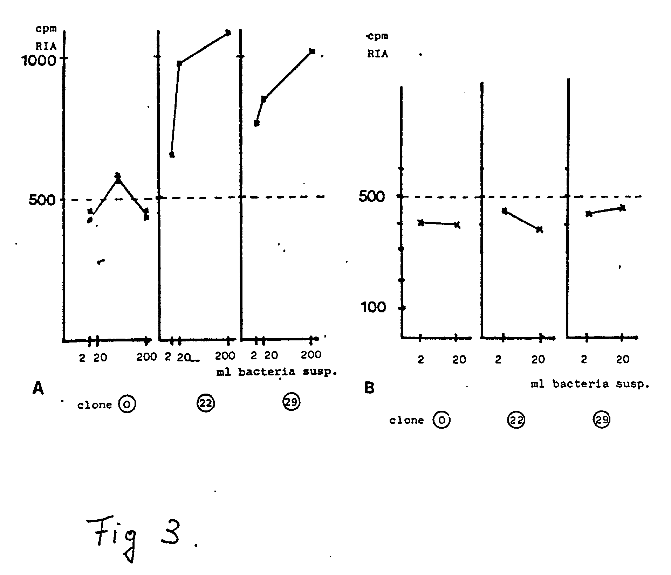

Fig. 3: Wasserschock-Lysate verschiedener Mengen von aliquoten Teilen einzelner Bakterienklone wurden einem HAV-Radioimmuntest unterworfen. A) Radioimmuntests mit Kugeln, die mit Anti-HAV-positivem menschlichem Serum umhüllt sind; B) mit Kugeln, die mit Anti-HAV-negativem menschlichem Serum umhüllt sind.

Für die Untersuchungen wurden HAV-Partikel aus einem Stuhl hergestellt, der von einem Patienten, bei dem sich vier Tage später typische Hepatitis A einstellte, erhalten worden war. Die Gesamtausbeute an Partikeln entsprach ungefähr einer Virusmasse von etwa 10 bis 20 µg. Aus den Partikeln extrahierte RNA wurde mit reverser Transcriptase des Myeloblastosis-Virus von Vögeln (AMV) in cDNA transkribiert, und die Analyse der cDNA durch Gel-Elektrophorese ergab eine ungefähre Ausbeute von 10 bis 50 ng DNA von uneinheitlicher Grösse mit der überwiegenden Population bei etwa 800 bis 1000 Basenpaaren. Die DNA wurde in den Pst I-Restriktionsort des Plasmids pBR 322 kloniert. E. coli X 1776 wurde dann durch die Hybridplasmide transformiert, und Klone wurden in Gegenwart von Tetracyclin selektiert. Aus einzelnen Bakterienklonen wurden Plasmide hergestellt. Die Analyse der klonierten Plasmide ergab, dass viele eine DNA-Einfügung (Fig. 1) im Bereich von 300 bis 800 Nucleotiden enthielten.

Um zu ermitteln, ob die DNA-Einfügung HAV-spezifische Nucleotidsequenzen aufwies, wurden die rekombinierten Plasmide zu HAV-spezifischer, mit $^{32}$P markierter RNA hybridisiert. Diese RNA-Proben wurden aus einer anderen Quelle hergestellt, nämlich aus mit HAV infizierten Zellkulturen, um falsche Ergebnisse als Folge einer Kontamination mit nicht-viraler RNA der für die Transkription verwendeten ursprünglichen, vom Stuhl gewonnenen viralen RNA zu vermeiden. Die meisten Klone, die Einfügungen enthielten, hybridisierten zu dieser HAV-RNA (Fig. 2).

Die Bakterienklone wurden auf Expression von Antigen nach Wasscherschock-Lysis der Bakterien (Villa-Komaroff et al, loc. cit.) untersucht, und die Schockfluids wurden in einem Festphasen-Radioimmuntest (HAVAB von Abbott Laboratories, North Chicago, USA) auf HAV-Antigene getestet. Zwei Klone waren positiv, d.h. sie reagierten auf einem 2- bis 3-fachen Niveau über dem negativen Hintergrund. Steigende Mengen von Schockfluids (2,20 bzw. 200 ml) banden proportional steigende Mengen von Radioaktivität beim Radioimmuntest (Fig. 3A).

Um diese Ergebnisse zu bestätigen, wurde der Radioimmuntest unter Verwendung einer festen Phase (Kugeln) durchgeführt, die mit HAV-negativem Serum umhüllt worden waren. Fig. 3B zeigt, dass kein Schockfluid unter diesen Bedingungen reagierte, ein weiterer Beweis, dass HAV-spezifische Polypeptide in den Klonen 22 und 29 exprimiert sind.

Die vorhandenen Klone können nunmehr als Hybridisierungssonden und Primer für die Herstellung von weiterer cDNA unter Verwendung von RNA von mit HAV infizierten Zellkulturen verwendet werden.

Reinigung von Viruspartikeln

HAV-Partikel wurden aus 25 g gefrorenem Stuhl gereinigt wie beschrieben (G. Siegl und G. G. Frösner, J. Virol. 26 (1978) 40 und 48). Die Partikel wurden, kurz gesagt, aus dem Stuhl entnommen, an einem vorgebildeten CsCl-Dichtegradienten (Dichte 1,25–1,50 g/cm$^3$, mittl. Dichte 1,35 g/cm$^3$), der oben ein Kissen von 30%iger Saccharose zur Absorption des grössten Teils der nicht-verwandten Proteine trug, gebandet, und die Fraktion mit einer Dichte von 1,34 g/cm$^3$ wurde durch Sedimentationszentrifugation über einen Saccharosegradienten gebandet. HAV-Antigen-positive Fraktionen, getestet mit dem Standard-Radioimmuntest der Feststoffphase, G.G. Frösner, Münch. med. Wschr. 119 (1977) 825 unter Verwendung von HAVAB-Test-Kits der Abbot Laboratories, North Chicago) wurden zusammengegossen und dann erneut mit einem CsCl-Dichtegradienten ähnlich wie oben gebandet. Die Gesamtausbeute von Partikeln aus dieser Zentrifugation band 10$^7$ cpm von mit I$^{125}$ markiertem Anti-HAV. Dies entspricht ungefähr einer Virusmasse von etwa 10 bis 20 ug.

Synthese von komplementärer DNA (cDNA)

RNA wurde aus den Partikeln nach der Guanidin-Rhodanid-Methode extrahiert (A. Ullrich, J. Shine, R. Chirgivin, E. Pictet, W.J. Rutter und H.M. Goodman, Science 196 (1977) 1313), so dass sichergestellt wurde, dass keine RNase-Aktivität vorhanden war. Sie wurde mit 100 U reverser Transcriptase des Myeloblastosis-Virus von Vögeln (Boehringer Mannheim) in Gegenwart von Oligo-d-T$_{12-18}$ als Primer, 8 mM MgAc$_2$, 0,01% NP-40®, Desoxydenosintriphosphat (dATP), Desoxyguanidintriphosphat (dGTP), Desoxythymidintriphosphat (dTTP) je 1 mM, 0,2 mM Desoxycytidintriphosphat (dCTP) und 0,5 mCi $^{32}$P-dCTP als radioaktiver Nucleotidvorstufe (NEN) 2 Stunden bei 42 °C transkribiert. Das gesamte Inkubationsgemisch wurde 1 Minute bei 80 °C erhitzt und schnell gekühlt, worauf erneut zur Doppelstrang-Synthese 50 U reverse Transcriptase zugesetzt wurden und das Gemisch 2 Stunden bei 30 °C inkubiert wurde. Die Analyse der komplementär-DNA (cDNA) durch Gelelektrophorese ergab eine ungefähre Ausbeute von 10–50 ng DNA einer uneinheitlichen Grösse zwischen einigen Hundert bis zu 2000 Basenpaaren, wobei die grössere Population bei etwa 800 bis 1000 Basenpaaren lag.

Klonieren von cDNA in Plasmide

An die DNA wurde das Desoxycitidin am 3'-Ende (T. Nelson und D. Brutlag, Methods of Enzymology 68 (1979) 41, herausgegeben von R. Wu (Academic Press New York) unter Verwendung von terminaler Transferade (P.-L. Biochemicals Inc., Milwaukee) angehängt und dann mit Plasmid-pBR 322-DNA hybridisiert, die mit den Restriktionsenzymen PstI durchschnitten worden war, und an deren 3'-Enden Desoxyguanidin (dG)-Homopolymere angehängt worden waren (Nelson und Brutlag, loc. cit.). E. Coli X 1776 wurde dann mit den Hybrid-Plasmiden transformiert und Klone wurden in L-Nährmedium in Gegenwart von Tetracyclin (12 µg/cm$^3$) selektiert (H.M. Goodman und R.J. MacDonalt, Methods of Enzymology 68 (1979) 75, Herausgeber R. Wu (Academic Press, New York).

Analyse von Plasmid-Einfügungen

Einzelne bakterielle Klone wurden bis zu 100 ml in Gegenwart von Uridin gemäss M.V. Norgard, K. Emingholz und J. Monahan (J. Bact. 138 )1979), 270) gezüchtet und Plasmide wurden durch Lysis mit Triton X-100® und anschliessende CsCl-Sedimentationszentrifugation in Gegenwart von Ethidiumbromid hergestellt (Villa-Komaroff, L., A. Efstradiadis, S. Broome, P. Lomedico, R. Tizard, S.P. Naber, W.L. Chick und W. Gilbert, Proc. Natl. Acad. Sci. USA 75 (1978) 3727 und F. Bolivar und K. Backman, Methods of Enzymology 68 (1979) 245, Herausgeber R. Wu (Academic Press, New York). Die Grösse der Plasmide wurde durch Elektrophorese an 1%igem Agarosegel analysiert, und die Banden wurden mit Plasmid-Markern mit DNA-Einfügungen von bekannter Grösse verglichen.

Hybridisierung von HAV-RNA zu Plasmid-DNA

Einzelne Bakterienklone wurden auf Nitrocellulose-Filtern über Agar gemäss M. Grundstein und D.S. Hogness (Proc. Natl. Acad. Sci. USA 72 (1975) 3691) gezüchtet. HAV-spezifische RNA wurde von den mit HAV infizierten PLC/PRF5-Zellen gewonnen (J. Alexander, G. Macnab, R. Saunders, Perspectives in Virology 10 (1978) 103 (Herausgeber M. Pollard (Raven Press, New York). Vier Wochen nach der Infektion mit HAV (Frösner et al, 1979, loc. cit.; Deinhardt et al, 1981, loc. cit.), wurde den Zellen das Phosphat für 12 Std. entzogen, worauf sie mit $^{32}$PO$_4$ (NEN; 1 ci/cm$^3$, trägerfrei (24 Std. lang markiert und lysiert wurden. Die HAV-Partikel wurden durch CsCl-Sedimentationszentrifugation gereinigt. Eine Bande mit der Dichte von HAV (1,34 g/cm$^3$), die bei einem HAV-Radioimmuntest eine positive Reaktion zeigte, wurde mit Phenol behandelt, und die RNA (100 000 cpm/Filter) wurde in 50%igem Formamid, 5 × SSC bei 40 °C für 15 Stunden zu den auf Nitrocellulosefiltern gezüchteten Bakterienklonen hybridisiert. Jedes Filter enthielt einen negativen Kontroll-Klon mit dem Plasmid pBR 322, der keine Einfügung hatte.

Sequenzieren der Hybrid-DNA

Die Hybrid-DNA der positiven Klone können mit der Endodesoxiribonuclease aus der PstI Stelle des Plasmidvektors ausgeschnitten werden und deren Nucleotidsequenz kann nach den Methoden von A.M. Maxam, Walter Gilbert (Proc. Natl. Acad. Sci. USA 74, 560, (1977)) oder F. Sanger, S. Nicklen und A.R. Coulson (Proc. Natl. Acad. Sci. USA 74, 5463 (1977)) ermittelt werden.

Expression von HAV-Antigen

Einzelne Bakterienklone wurden in jeweils 250 ml Suspension bis zu einer optischen Dichte (O.D.) von 0,8 gezüchtet, worauf aliquote Teile von je 2, 20 und 200 ml zentrifugiert wurden. Die Bakterien-Pellets wurden mit Wasser gemäss Villa-Komaroff et al (1978, loc. cit.) lysiert, und aliquote Teile von 300 µl jedes Wasserschockfluids wurden auf HAV-Antigene in einem Festphasen-HAV-Radioimmuntest (siehe oben) untersucht. Als Kontrolle wurden die beim Radioimmuntest verwendeten Polystyrolkugeln mit Anti-HAV Negativserum umhüllt.

**Patentanspruch**

Verfahren zur Herstellung von Hybridisierungssonden zur Selektion von HAV-cDNA enthaltenden Klonen, die für HAV Antigene codieren, dadurch gekennzeichnet, dass man

a) doppelsträngige DNA, die durch reverse Transkription der viralen RNA des HAV hergestellt wurden und überwiegend eine Grösse von etwa 800 bis 1000 Basenpaaren hat, in der PstI Stelle des Plasmids pBR322 kloniert;

b) E. coli X 1776 mit den in a) entstandenen Hybrid-Plasmiden transformiert und in Gegenwart von Tetracyclin die Plasmid-haltigen Klone selektiert;

c) die mit $^{32}$P markierten HVA-RNA hybridisierenden Klone weiter selektiert; und

d) die Bakterienklone auf Expression von HAV-Antigen nach Wasserschock-Lysis der Bakterien durch einen Festphasen-Radioimmunotest auf HAV-Antigene testet und diejenigen auswählt, die auf einem 2- bis 3-fachen cpm-Wert über dem negativen Testhintergrund reagieren.

**Claim**

A process for the preparation of hybridization probes for the selection of HAV-cDNA-containing clones coding for HAV antigens, characterized in that

a) double-stranded DNA prepared by reverse transcription of the viral RNA of HAV and predominantly having a size of about from 800 to 1000 base pairs is cloned in the Pst I site of the plasmide pBR322;

b) E. coli X 1776 is transformed with the hybrid plasmides formed in a) and the plasmid-containing clones are selected in the presence of tetracycline;

c) the $^{32}$P-labelled HVA-RNA hybridizing clones are further selected; and

d) the bacteria clones are tested for expression of HAV antigen after water-shock lysis of the bacteria by means of a solid phase radioimmunoassay for HAV antigens, and those are chosen which react on 2- to 3-fold cpm level over the negative test background.

**Revendication**

Procédé pour la préparation de sondes d'hybridisation en vue de la sélection de clones contenant HAV-cDNA qui codent pour les antigènes HAV, caractérisé en ce que:

a) on clone, au site Pst I du plasmide pBR 322, le DNA double brin qui a été produit par transcription inverse du RNA viral du HAV et possède en prédominance une longueur approximative de 800 à 1000 paires de base;

b) on transforme E. coli 1776 avec les plasmides hybrides (recombinants) obtenus en a) et on sélectionne les clones contenant le plasmide en présence de tétracycline;

c) on sélectionne encore les clones d'hybridation HAV-RNA marqués au $^{32}$p; et

d) on teste les clones bactériens pour l'expression d'antigène-HAV, après lyse avec choc osmotique des bactéries, par un radioimmuno-essai en phase solide sur les antigènes HAV et on sélectionne ceux qui réagissent à une valeur de cpm deux à trois fois supérieure à la valeur négative de bruit de fond du test.

plasmid
+ insertion
plasmid

a   b   c   d

Fig 1

A

B

Fig. 2

Fig 3.